# EUROPEAN PATENT SPECIFICATION

(11) **EP 2 471 489 B1**
(45) Date of publication and mention of the grant of the patent: **11.07.2018**
(21) Application number: 11275165.6
(22) Date of filing: 21.12.2011
(51) Int. Cl.: A61F 2/90, A61F 2/07, A61F 2/06, D03D 13/00, D03D 15/00

(54) **Composite woven fabric for endoluminal devices**
Verbundstoffgewebe für endoluminale Vorrichtungen
Étoffe tissée composite pour dispositifs endoluminaux

(30) Priority: 30.12.2010 US 201061428569 P
(43) Date of publication of application: 04.07.2012
(73) Proprietor: Cook Medical Technologies LLC, Bloomington, IN 47402-0489 (US)
(72) Inventor: Rasmussen, Erik E., DK-4200 Slagelse (DK); Dierking, William, Louisville, KY Kentucky 40222 (US); Kuppurathanam, Shyam, Bloomington, IN Indiana 47403 (US)
(74) Representative: Georgiou, Sarah Caroline

(56) References cited:
- EP-A2- 2 136 858
- WO-A1-2010/139340
- WO-A2-02/28314
- US-A1- 2009 171 440

## Description

### TECHNICAL FIELD

The present invention relates to woven fabrics. More particularly, the present invention relates to low profile woven fabrics for implantable medical devices constructed of at least two different materials having different characteristics.

### BACKGROUND

This invention relates generally to medical devices and particularly to medical devices that are implantable within the human or animal body for the repair of damaged vessels, ducts or other physiological passageways and cavities.

The physiological passageways and cavities of human and animal bodies, for example, blood vessels and ducts, occasionally weaken or even rupture. One common surgical intervention for weakened, aneurysmal or ruptured passageways or ducts involves the use of an endoluminal prosthesis to provide some or all of the functionality of the original, healthy passageway or duct and/or preserve any remaining vascular integrity by replacing a length of the existing passageway or duct wall that spans the site of failure or defect. Endoluminal prostheses may be of a unitary construction or may be comprised of multiple prosthetic modules.

EP 2136858 describes a type of woven fabric for implantable medical devices. WO2010/139340 describes a medical device comprising yarns of a shape memory material and polymer yarns, wherein the yarns made of shape memory material have a polymer sheathing.

### BRIEF SUMMARY

In one aspect, a composite woven fabric for a low profile implantable medical device having a plurality of textile strands of a first material aligned in a first direction interlaced with a plurality of textile strands of a second material. The textile strands have a size between about 10 denier to about 20 denier (corresponding to about 11 to 22 dtex). The first material has at least one characteristic different from the second material. The second material may react more favorably with blood when placed within an artery. For example, the second material may show greater resistance to biodegradation within the blood vessels. The second material may promote thrombosis.

The yarns aligned in the first direction may comprise warp yarns. The yarns aligned in the second direction may comprise weft yarns. The composite material may have between about 50 and about 300 weft yarns per inch (corresponding to about 127 and about 762 weft yarns per cm) and between about 50 and about 300 warp yarns per inch (corresponding to about 127 and about 762 warp yarns per cm). The first material and/or the second material may comprise a polymer. The first material may comprise ultra-high molecular weight polyethylene. The second material may comprise polyethylene terephthalate.

The fabric may comprise a weave selected from the group consisting of a plain weave, a basket weave, a rep weave, a rib weave, a twill weave, a leno weave, a mock leno weave, a satin weave, a double weave, or a variation thereof. Preferably the fabric comprises a plain weave. The composite woven fabric may have a pick count between about 3 and about 500. The textile strands may have a size of 20 denier (corresponding to 22 dtex). The textile strands aligned in the first direction may have at least one float of at least two textile strands aligned in the second direction.

In another aspect, a woven fabric suitable for an implantable medical device having polyethylene terephthalate textile strands having a size between about 10 denier and about 20 (corresponding to about 11 and 22 dtex) denier aligned in a first direction interlaced with polyethylene strands having a size between about 10 denier to about 20 denier (corresponding to about 11 and 22 dtex) aligned in a second direction. The composite woven fabric may have a primary weave comprising a plain weave. The polyester textile strands in the first direction may have at least one characteristic different than the polyethylene textile strands aligned in the second direction.

The polyethylene textile strands aligned in the first direction comprise may warp strands. The polyester textile strands aligned in the second direction may comprise weft strands. The polyester textile strands may be polyethylene terephthalate. The woven fabric may have a pick count between about 3 and about 500. The polyester textile strands and the polyethylene textile strands may have a size of 20 denier (corresponding to 22 dtex). The polyethylene textile strands aligned in the first direction may have at least one float of at least two polyester textile strands aligned in the second direction.

In yet another aspect, a method of producing a woven fabric for an implantable medical device. The method comprises providing textile strands of a first material to be aligned in a first direction and providing textile strands of a second material to be aligned in a second direction. The first material has at least one characteristic different from the second material. The textile strands are woven to produce a woven graft. The textile strands may have a size between about 10 denier to about 20 denier (corresponding to about 11 to 22 dtex). In one aspect, the textile strands are woven in a tubular double weave.

The first material and the second material may have a size of 20 denier (corresponding to 22 dtex). At least one of the textile strands in the first direction may be woven to have at least one float of at least two textile strands in the second direction. The textile strands aligned in the first direction may comprise warp strands and the textile strands aligned in the second direction may comprise weft strands. The first material may be polyethylene. The second material may be polyethylene terephthalate.

### BRIEF DESCRIPTION OF THE DRAWINGS

Embodiments of the present invention are described below, by way of example only, with reference to the accompanying drawings, in which:
FIG. 1 is a schematic representation of an embodiment of woven fabric weave pattern.
FIG. 2 is a schematic representation of another woven fabric weave pattern.
FIG. 3 is a schematic representation of yet another fabric weave pattern.
FIG. 4 is a perspective illustration of one embodiment of a medical device comprising a woven fabric of the present invention.

The components in the drawings are not necessarily to scale, emphasis instead being placed upon illustrating the principles of the teachings herein.

### DETAILED DESCRIPTION

Unless defined otherwise, all technical and scientific terms used herein have the same meaning as commonly understood to one of ordinary skill in the art to which this invention belongs.

The term "implantable" refers to an ability of a medical device to be positioned at a location within a body, such as within a body lumen.

The term "strand" as used herein is a generic term for a continuous strand of material suitable for weaving. For example, strands may include, but are not limited to monofilaments, filaments twisted together, fibers spun together or otherwise joined, yarns, roving yarns, crepe yarns, ply yarns, cord yarns, threads, strings, filaments laid together without twist, as well as other configurations.

The term "binding point" refers to the intersection of a single strand in a first direction with strands in a second direction. For example, a strand in a first direction may run "over" one or multiple strands in a second direction, have a binding point, and run "under" one or more subsequent strands in the second direction.

The term "float" refers to that portion of a strand in a first direction that extends over or under two or more strands in a second direction without a binding point. For example, a strand in a first direction may have a binding point with strands in a second direction, then float over 5 adjacent strands in the second direction, and then have another binding point with strands in the second direction.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "endoluminal" refers to or describes the internal or inside of a lumen, duct, and other passageways or cavities located in a human or other animal body. A lumen or a body passageway may be an existing lumen or a lumen created by surgical intervention. As used in this specification, the terms "lumen" or "body passageway," and "vessel" are intended to have a broad meaning and encompass any duct (e.g., natural or iatrogenic) or cavity within the human body and may include without limitation, blood vessels, respiratory ducts, gastrointestinal ducts, such as the biliary duct, intestines, the esophagus, the pericardial cavity, the thoracic cavity, the pericadial cavity, and the like. Accordingly, the terms "endoluminal device" or "endoluminal prosthesis" describe devices that can be placed inside or moved through any such lumen or duct.

The terms "patient," "subject," and "recipient" as used in this application may refer to any animal, particularly humans.

The term "about" used with reference to a quantity includes variations in the recited quantity that are equivalent to the quantity recited, such as an amount that is insubstantially different from a recited quantity for an intended purpose or function.

The term "graft" or "graft material" describes an object, device, or structure that is joined to or that is capable of being joined to or implanted in or against a body part to enhance, repair, or replace a portion or a function of that body part. A graft by itself or with the addition of other elements, such as structural components, may constitute an endoluminal prosthesis. The graft may be comprised of a single material, a blend of materials, a weave, a laminate, or a composite of two or more materials. The graft may also be constructed from a synthetic, for example and without limitation, a polymer. The graft may be formed from a single layer or multiple layers of material. In embodiments employing a plurality of layers of material, the layers may remain separate, or may be attached to each other through a secondary process such as sintering, curing, adhesives, and sutures or the like.

Studies show that, with endovascular grafts, the major component contributing to the volume of the delivery system is the graft material. The present invention relates to low profile woven fabrics for implantable medical devices constructed of at least two different materials having different characteristics. The woven fabric permits fabrication of a device having a lower profile and is capable of delivery through a low profile delivery device of preferably 12 Fr or less. In one embodiment, the woven fabric comprises weaving low denier polyethylene terephthalate fibers, commonly known as PET, with low denier polyethylene fibers to achieve a thin woven fabric that may be used to provide a low profile, durable, biocompatible endovascular graft having the strength and abrasion resistant characteristics of PE with the high biocompatibility of PET.

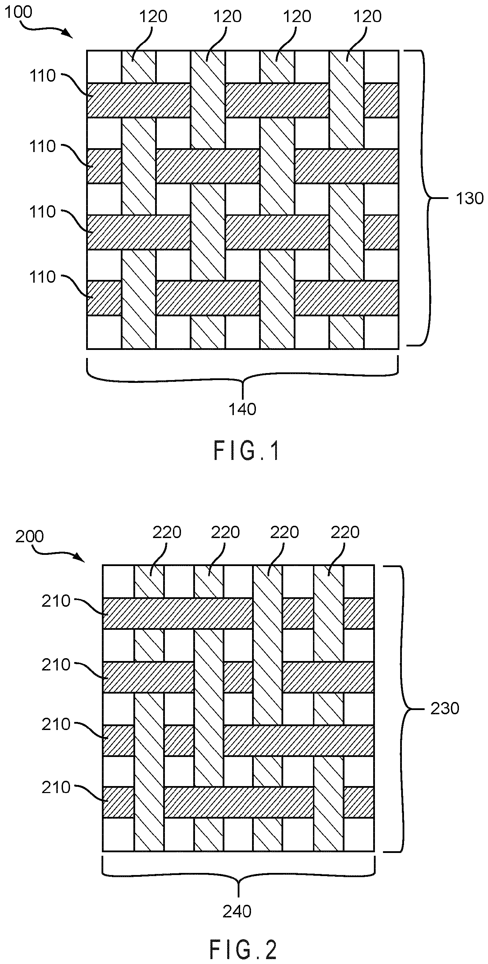

FIG. 1 illustrates an embodiment of a composite woven fabric 100 having a plurality of textile strand fibers of a first material 110 aligned in a first direction 130 interwoven with a plurality of textile strand fibers of a second material 120 in a second direction 140. The composite woven fabric 100 may be composed of two or more materials and may be natural, synthetic, or manufactured. In some aspects, the first material 110 and the second material 120 of the composite woven fabric 100 may have different characteristics. Preferably, the textile strands are formed from polymers. For example, biocompatible materials from which textile strands can be formed include, but are not limited to, polyesters, such as poly(ethylene terephthalate), and polyethylene. Even more preferably, the textile strands comprise polyethylene terephthalate and ultra-high molecular polyethylene. A preferred commercial example of polyethylene terephthalate especially suited for weaving is Dacron®. A preferred commercial example of ultra-high molecular polyethylene suited for weaving is Dyneema®.

As shown in FIG. 1, the textile strands 110, 120 are woven, for example, in a plain weave characterized by a regular 1/1 interlacing of strands. That is, each strand aligned in the first direction (e.g. warp direction) move alternatively over and under adjacent strands aligned in a second direction (e.g., weft direction). This plain weave pattern produces the maximum number of binding points, and is thus, a firm, durable weave. However, the woven composite fabric material may comprise any kind of weave. For example, the woven fabric may include, but is not limited to, weaves such as plain weaves, basket weaves, rep or rib weaves, twill weaves (e.g., straight twill, reverse twill, herringbone twill), satin weaves, and double weaves (e.g., double-width, tubular double weave, reversed double weave). The composite woven fabric material may be woven in any suitable manner. For example, the fabric may be woven on a table loom, a floor loom, a jacquard loom, a counterbalance loom, a jack loom, or an upright loom. Desirably, the fabric is woven on a floor loom.

The spacing of the strands within the weave is expressed in terms of a linear density or line density of strands, and may depend on the denier of the strands. A higher linear density is indicative of a smaller spacing between adjacent strands. During the weaving process to create the composite woven fabric, the sett and pick count are kept constant. The sett may be between about 50 and about 300 ends per inch (corresponding to about 127 and 762 ends per cm) and the pick count may be between about 3 and about 500 (corresponding to about 8 and about 1270 picks per cm) picks per inch. An "end" refers to an individual warp yarn, and a "pick" refers to an individual weft yarn. In some aspects, the composite woven fabric may consist of a balanced weave (e.g. the composite woven fabric has the same number of weft yarns per inch as warp yarns per inch). In other aspects, the composite woven fabric has an unbalanced weave (e.g. the composite woven fabric has an unequal distribution of warp and weft yarns, with one or the other predominating). For example, a composite woven fabric for producing a prosthesis, such as a stent graft, may comprise a plain weave having 150 ends per inch (corresponding to 381 per cm) and 250 picks per inch (corresponding to 635 per cm).

Determination of which combination of materials in which direction of the fabric is most appropriate may be based on a variety of factors, including intended clinical application, desired properties of the woven fabric, weave type, and strand properties such as the size or denier of the strand and the shape of the strands. The composite woven fabric preferably has a thickness that is reduced compared to conventional woven fabrics. A thickness reduction may be achieved by the use of a smaller denier and/or a reduced density of the weave. In this aspect, textile strands in the woven fabric range in size from about 10 denier and about 20 denier (corresponding to about 11 to 22 dtex).

During the weaving process, textile strands woven in the weft direction are subjected to much lower tensile loads than textile strands woven in the warp direction. Thus, in order to use smaller denier yarns in the woven graft material, the first material 110 has sufficient tensile strength to sustain the tensile loads caused in the warp direction during weaving. In addition, because the tensile loads are lower in the weft direction, a small denier fiber having certain favorable characteristics may be able to be used in the weft direction. In some aspects, polyethylene fibers are woven in the warp direction, while polyethylene terephthalate fibers are woven in the weft direction of the woven fabric. Polyethylene textile strands are suitable in the warp direction because of its overall strength characteristics, low surface energy, and ability to repel most biologic material. Polyethylene terephthalate are suitable in the weft direction due to its favorable reaction to blood within the artery of a patient when the woven material is used for a prosthesis, such as a stent graft.

The combination of polyethylene terephthalate fibers in the warp direction along with polyethylene fibers in the weft direction provides the composite fabric material with substantial advantages over conventional graft materials. In particular, the tensile strength of polyethylene allows one to maintain strength characteristics of conventional woven fabric materials in the end product using smaller denier fibers. Further, the composite woven fabric provides geometric advantages by providing the necessary reduction in graft material thickness to achieve smaller delivery system diameters for low profile endovascular graft material. Moreover, the composite woven graft material utilizes the favorable biological response of PET as a blood contacting material and subsequent pressure barrier for an endovascular graft.

FIG. 2 discloses an alternative embodiment of the composite woven fabric 200 having textile strands of a first material 210 in a first direction 230 and textile strands of a second material 220 in a second direction 240. The textile strands are woven in a 2/2 textile weave pattern. As shown, the binding points of the weft yarns and the warp yarns occur in a diagonal progression known as a wale. Floats appear in both the first direction 230 and the second direction 240. Twill weaves are described as balanced when the same number of textile strands in the first 230 and second 240 directions intersect. This weaving pattern is prepared by passing the textile strands of the first material 210 in the first, or weft, direction 230 over one or more textile strands of the second material 220 in the second, or warp, direction 240 and then under two or more warp threads and so on, with a "step" or offset between rows to create the characteristic diagonal pattern.

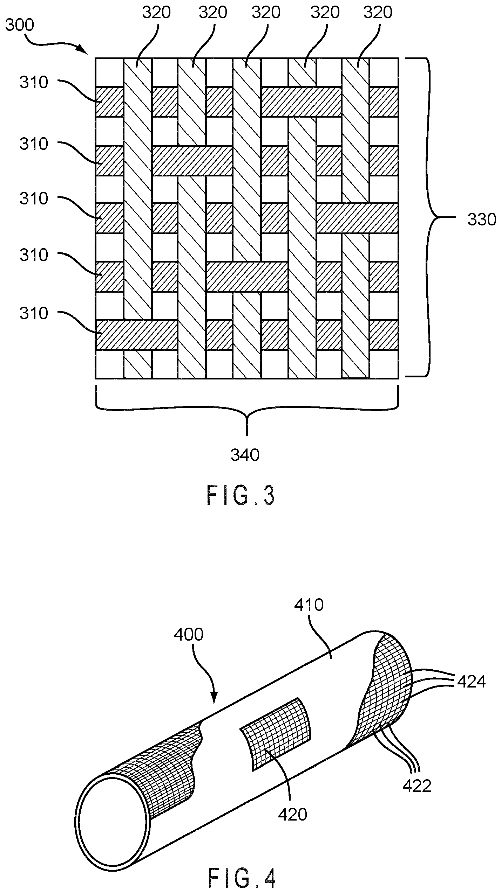

FIG. 3 depicts a further embodiment of the composite woven fabric 300 having textile strands of a first material 310 in a first direction 330 and textile strands of a second material 320 in a second direction 340. The textile strands are woven in a 5-Harness satin weave. As shown, the satin weave is characterized by four or more textile strands of the first material 310 in the first, or weft, direction 330 floating over textiles strands of the second material 320 in the second, or warp direction 340.

The fabric of the present invention is suitable for use in a variety of implantable or insertable medical devices, for example surgically or endoluminally, of any shape or configuration comprising woven fabric. The medical device may be any device comprising a woven fabric that is introduced temporarily or permanently into the body for the treatment of a medical condition. For example, such medical devices may include, but are not limited to endovascular grafts, stent grafts, balloon catheters, meshes, vascular grafts, stent-graft composites, filters (e.g., vena cava filters), vascular implants, tissue scaffolds, myocardial plugs, valves (e.g., venous valves), various types of dressings, endoluminal prostheses, vascular supports, or other known biocompatible devices.

The medical device may be balloon-expandable or, preferably, self-expanding and may be a bifurcated integrated stent-graft configured for any blood vessel including coronary arteries and peripheral arteries (e.g., renal, superficial femoral, carotid, and the like), a urethral integrated stent-graft, a gastrointestinal integrated stent-graft, or an esophageal integrated stent-graft, for example. Typical subjects (also referred to herein as "patients") are vertebrate subjects (i.e., members of the subphylum cordata), including, mammals such as cattle, sheep, pigs, goats, horses, dogs, cats and humans.

FIG. 4 is a schematic of a stent graft 400 having a tubular structure 410 and a woven fabric 420. The woven fabric 420 has circumferential strands 422 and longitudinal strands 424, though the strands need not be radial and longitudinal and may have any possible orientation. The circumferential strands 422 and the longitudinal strands 424 may have any suitable weave configuration, such as those shown by FIG. 1-3. Although two dimensional weave patterns are shown, it is possible for the composite woven fabric to have strands woven in a third direction. The stent graft may include stents made from numerous metals and alloys. In one example, the stents comprise a shape-memory material such as a nickel-titanium alloy ("Nitinol"). Moreover, the structure of the stents may be formed in a variety of ways to provide a suitable support structure. For example, one or more stents may be made from a woven wire structure, a laser-cut cannula, individual interconnected rings, or another pattern or design. The stents may be configured in the form of one or more "Z-stents", each of which may comprise a series of substantially straight segments interconnected by a series of bent segments. The bent segments may comprise acute bends or apices. The stents are arranged in a zigzag configuration in which the straight segments are set at angles relative to each other and are connected by a bent segment. However, as noted above, the stents may comprise any suitable configuration and one or more stents may be provided.

Throughout this specification various indications have been given as to preferred and alternative embodiments of the invention. However, the foregoing detailed description is to be regarded as illustrative rather than limiting and the invention is not limited to any one of the provided embodiments. It should be understood that it is the appended claims that are intended to define the invention.

## Claims

1. A composite woven fabric (100, 200, 300, 420) for a low profile implantable medical device: comprising a plurality of textile strands of a first material (110, 210, 310, 422) aligned in a first direction (130, 230, 330) interlaced with a plurality of textile strands of a second material (120, 220, 320, 424), where the textile strands have a size between 10 denier to 20 denier (corresponding to 11 to 22 dtex), and where the first material (110, 210, 310, 422) has at least one characteristic different from the second material (120, 220, 320, 422), and where the first material comprises ultra-high molecular weight polyethylene and the second material comprises polyethylene terephthalate.

2. The composite woven fabric of claim 1, where the yarns aligned in the first direction (130, 230, 330) comprise warp yarns, and the yarns aligned in the second direction (140, 240, 340) comprise weft yarns.

3. The composite woven fabric of any preceding claim, where the second material (120, 220, 320, 422) comprises between 50 and 300 weft yarns per inch (corresponding to 127 and 762 per cm) and between 50 and 300 warp yarns per inch (corresponding to 127 and 762 per cm).

4. The composite woven fabric of any preceding claim, where the fabric comprises a weave selected from the group consisting of a plain weave, a basket weave, a rep weave, a rib weave, a twill weave, a leno weave, a mock leno weave, a satin weave, a double weave, or a variation thereof, preferably a plain weave.

5. The composite woven fabric of any preceding claim, where the textile strands have a size of 20 denier (corresponding to 22 dtex).

6. The composite woven fabric of any preceding claim, where the textile strands aligned in the first direction (130, 230, 330) have at least one float of at least two textile strands aligned in the second direction (140, 240, 340).

7. A method of producing a composite woven fabric (100, 200, 300, 420) for an implantable medical device comprising the steps of:
providing a plurality of textile strands of a first material (110, 210, 310, 422) to be aligned in a first direction (130, 230, 330); and
providing a plurality of textile strands of a second material (120, 220, 320, 424) to be aligned in a second direction (140, 240, 340), the first material having at least one characteristic different from the second material; and
weaving the textile strands to produce a woven fabric;
where the textile strands have a size between 10 denier to 20 denier (corresponding to 11 dtex to 22 dtex), and where the first material comprises ultra-high molecular weight polyethylene and the second material comprises polyethylene terephthalate.

8. The method of claim 7, where at least one of the textile strands in the first direction (130, 230, 330) is woven to have at least one float of at least two textile strands in the second direction (140, 240, 340).

9. The method of any of claim 7-8, where the textile strands aligned in the first direction (130, 230, 330) comprise warp strands and the textile strands aligned in the second direction (140, 240, 340) comprise weft strands.

## Patentansprüche

1. Verbundstoffgewebe (100, 200, 300, 420) für eine implantierbare medizinische Vorrichtung mit niedrigem Profil:
aufweisend mehrere Textilfäden eines ersten Materials (110, 210, 310, 422), die in einer ersten Richtung (130, 230, 330) ausgerichtet sind, verwoben mit mehreren Textilfäden eines zweiten Materials (120, 220, 320, 424), wobei die Textilfäden eine Größe zwischen 10 Denier und 20 Denier (entsprechend 11 bis 22 dtex) aufweisen, und wobei das erste Material (110, 210, 310, 422) mindestens ein Kennzeichen aufweist, das vom zweiten Material (120, 220, 320, 422) abweicht, und wobei das erste Material Polyethylen mit ultrahohem Molekulargewicht aufweist und das zweite Material Polyethylenterephthalat aufweist.

2. Verbundstoffgewebe nach Anspruch 1, wobei die Garne, die in der ersten Richtung (130, 230, 330) ausgerichtet sind, Kettgarne aufweisen und die Garne, die in der zweiten Richtung (140, 240, 340) ausgerichtet sind, Schussgarne aufweisen.

3. Verbundstoffgewebe nach einem der vorhergehenden Ansprüche, wobei das zweite Material (120, 220, 320, 422) zwischen 50 und 300 Schussgarne pro Zoll (entsprechend 127 und 762 pro cm) und zwischen 50 und 300 Kettgarne pro Zoll (entsprechend 127 und 762 pro cm) aufweist.

4. Verbundstoffgewebe nach einem der vorhergehenden Ansprüche, wobei das Gewebe eine Webart aufweist, die aus der Gruppe ausgewählt ist, welche aus einer Grundbindung, einer Würfelbindung, einer Ripsbindung, einer Körperbindung, einer Dreherbindung, einer Scheindreherbindung, einer Satinbindung, einer Doppelbindung oder einer Variation davon besteht, vorzugsweise aus einer Grundbindung.

5. Verbundstoffgewebe nach einem der vorhergehenden Ansprüche, wobei die Textilfäden eine Größe von 20 Denier (entsprechend 22 dtex) aufweisen.

6. Verbundstoffgewebe nach einem der vorhergehenden Ansprüche, wobei die Textilfäden, die in der ersten Richtung (130, 230, 330) ausgerichtet sind, mindestens eine Flottierung von mindestens zwei Textilfäden aufweisen, die in der zweiten Richtung (140, 240, 340) ausgerichtet sind.

7. Verfahren zum Erzeugen eines Verbundstoffgewebes (100, 200, 300, 420) für eine implantierbare medizinische Vorrichtung, die folgenden Schritte aufweisend:
Vorsehen von mehreren Textilfäden eines ersten Materials (110, 210, 310, 422) zur Ausrichtung in einer ersten Richtung (130, 230, 330); und
Vorsehen von mehreren Textilfäden eines zweiten Materials (120, 220, 320, 424) zur Ausrichtung in einer zweiten Richtung (140, 240, 340), wobei das erste Material mindestens ein Kennzeichen aufweist, das vom zweiten Material abweicht; und Weben der Textilfäden zum Erzeugen eines Gewebes;
wobei die Textilfäden eine Größe zwischen 10 Denier und 20 Denier (entsprechend ungefähr 11 dtex bis 22 dtex) aufweisen, und wobei das erste Material Polyethylen mit ultrahohem Molekulargewicht aufweist und das zweite Material Polyethylenterephthalat aufweist.

8. Verfahren nach Anspruch 7, wobei mindestens einer der Textilfäden in der ersten Richtung (130, 230, 330) derart gewoben wird, dass er mindestens eine Flottierung von mindestens zwei Textilfäden in der zweiten Richtung (140, 240, 340) aufweist.

9. Verfahren nach einem der Ansprüche 7-8, wobei die Textilfäden, die in der ersten Richtung (130, 230, 330) ausgerichtet sind, Kettfäden aufweisen und die Textilfäden, in der zweiten Richtung (140, 240, 340) ausgerichtet sind, Schussfäden aufweisen.

## Revendications

1. Tissu tissé composite (100, 200, 300, 420) pour un dispositif médical implantable à profil bas :
comprenant une pluralité de brins textiles constitués d'un premier matériau (110, 210, 310, 422) alignés dans une première direction entrelacés avec une pluralité de brins textiles (130, 230, 330) constitués d'un deuxième matériau (120, 220, 320, 424), dans lequel les brins textiles ont une grosseur de 10 deniers à 20 deniers (correspondant à 11 à 22 dtex), et dans lequel le premier matériau (110, 210, 310, 422) a au moins une caractéristique différente du deuxième matériau (120, 220, 320, 422), et dans lequel le premier matériau comprend un polyéthylène à ultra-haute masse moléculaire et le deuxième matériau comprend du téréphtalate de polyéthylène.

2. Tissu tissé composite selon la revendication 1, dans lequel les fils alignés dans la première direction (130, 230, 330) forment des fils de chaîne, et les fils alignés dans la deuxième direction (140, 240, 340) forment des fils de trame.

3. Tissu tissé composite selon l'une quelconque des revendications précédentes, dans lequel le deuxième matériau (120, 220, 320, 422) comprend entre 50 et 300 fils de trame par pouce (correspondant à 127 et 762 par cm) et entre 50 et 300 fils de chaîne par pouce (correspondant à 127 et 762 par cm).

4. Tissu tissé composite selon l'une quelconque des revendications précédentes, le tissu comprenant un tissage sélectionné dans le groupe constitué d'une armure toile, d'un tissé natté, d'une armure reps, d'une armure côte, d'une armure sergé, d'une armure gaze, d'une armure fausse gaze, d'une armure satinée, d'un tissage double, ou d'une variante de ceux-ci, préférablement une armure toile.

5. Tissu tissé composite selon l'une quelconque des revendications précédentes, dans lequel les brins textiles ont une grosseur de 20 deniers (correspondant à 22 dtex).

6. Tissu tissé composite selon l'une quelconque des revendications précédentes, dans lequel les brins textiles alignés dans la première direction (130, 230, 330) comportent au moins un flotté constitué d'au moins deux brins textiles alignés dans la deuxième direction (140, 240, 340).

7. Procédé de fabrication d'un tissu tissé composite (100, 200, 300, 420) pour un dispositif médical implantable, comprenant les étapes qui consistent à :
fournir une pluralité de brins textiles constitués d'un premier matériau (110, 210, 310, 422) à aligner dans une première direction (130, 230, 330) ; et
fournir une pluralité de brins textiles constitués d'un deuxième matériau (120, 220, 320, 424) à aligner dans une deuxième direction (140, 240, 340), le premier matériau ayant au moins une caractéristique différente du deuxième matériau; et
tisser les brins textiles pour produire un tissu tissé,
dans lequel les brins textiles ont une grosseur de 10 deniers à 20 deniers (correspondant à 11 dtex à 22 dtex), et dans lequel le premier matériau comprend un polyéthylène à ultra-haute masse moléculaire et le deuxième matériau comprend du téréphtalate de polyéthylène.

8. Procédé selon la revendication 7, dans lequel au moins un des brins textiles dans la première direction (130, 230, 330) est tissé de façon à comporter au moins un flotté constitué d'au moins deux brins textiles dans la deuxième direction (140, 240, 340).

9. Procédé selon l'une quelconque des revendications 7 et 8, dans lequel les brins textiles alignés dans la première direction (130, 230, 330) forment des fils de chaîne et les brins textiles alignés dans la deuxième direction (140, 240, 340) forment des fils de trame.
